# Europäisches Patentamt

**European Patent Office**

**Office européen des brevets**

(19)

(11) Veröffentlichungsnummer: **0 183 061**
**B1**

(12)

# EUROPÄISCHE PATENTSCHRIFT

(45) Veröffentlichungstag der Patentschrift:
**16.03.88**

(51) Int. Cl.⁴: **C 07 C 125/067**

(21) Anmeldenummer: **85113568.1**

(22) Anmeldetag: **25.10.85**

(54) **Verfahren zur Herstellung von-(1-Methoxy-2-chlor)-ethoxyphenyl-N-methylcarbamat.**

(30) Priorität: **10.11.84 DE 3441108**

(43) Veröffentlichungstag der Anmeldung:
**04.06.86 Patentblatt 86/23**

(45) Bekanntmachung des Hinweises auf die Patenterteilung:
**16.03.88 Patentblatt 88/11**

(84) Benannte Vertragsstaaten:
**AT BE CH DE FR GB IT LI NL SE**

(56) Entgegenhaltungen:
**DE-A-2 231 249**

(73) Patentinhaber: **BASF Aktiengesellschaft, Carl-Bosch- Strasse 38, D-6700 Ludwigshafen (DE)**

(72) Erfinder: **Reissenweber, Gernot, Dr.,
Drosselstrasse 15, D-6737 Boehl- Iggelheim (DE)**
Erfinder: **Kersten, Siegfried, Dr., Max- Slevogt-Strasse 21, D-6710 Frankenthal (DE)**
Erfinder: **Doehnert, Detlef, Dr., Lisztstrasse 117, D-6700 Ludwigshafen (DE)**

LIBER, STOCKHOLM 1988

## Beschreibung

Die Erfindung betrifft ein Verfahren zur Herstellung von o-(1-Methoxy-2-chlor)-ethoxyphenyl-N-methylcarbamat - einem insektiziden Wirkstoff - durch Umsetzung von Brenzcatechincarbonat mit Methylamin und anschließender Umsetzung des entstandenen Brenzcatechinmonomethylcarbamates mit 1,2-Dichlorethylmethylether in Gegenwart eines sekundären Amins und vorzugsweise eines Lösungsmittels.

Verfahren zur Herstellung von Brenzcatechincarbamaten durch Umsetzung von Brenzcatechincarbonat mit primären oder sekundären Aminen sind beispielsweise aus Liebigs Ann. Chem. 300, 135 (1898); ibid. 362, 205 (1949), Journal für prakt. Chemie 313, 626 (1971) sowie DE-OS 26 50 828 bekannt, vgl. auch Houben-Weyl, 4. Auflage, Band 8, S. 139/140. Auch ist eine Umsetzung von Brenzcatechincarbonat mit Methylamin zum entsprechenden Carbamat bei Anwesenheit eines tertiären Amins bekannt (DE-A-32 27 931).

Die Umsetzung von Brenzcatechinmonomethylcarbamat mit 1,2-Dichlorethylmethylether in Gegenwart eines tertiären Amins als HCl-Fänger ist in den DE-A-22 31 249 und 26 50 828 beschrieben.

Die bekannten Verfahren haben - namentlich für das Herstellen im technisch wirtschaftlichen Maßstab - eine Reihe von Nachteilen, was vor allem auf den Alkylierungsschritt zutrifft: Bei Verwendung der in DE-OS 26 50 828 beschriebenen tert. Amine als HCl-Fänger bei der Umsetzung von Brenzcatechincarbamat mit 1,2-Dichlorethylmethylether werden lange Reaktionszeiten benötigt. Wirtschaftlich vertretbarer Umsatz geht somit auf Kosten guter Raum-Zeit-Ausbeute.

Auch sind die erzielten Ausbeuten nicht besonders hoch und die Reinheit und Stabilität des Endproduktes unbefriedigend.

Die Aufgabe, ein glatt verlaufendes Verfahren zur Herstellung von o-(1-Methoxy-2-chlor)-ethoxyphenyl-N-methylcarbamat zu finden, wird erfindungsgemäß dadurch gelöst, daß man sowohl die Umsetzung von Brenzcatechincarbonat mit Methylamin als auch die nachfolgende Umsetzung des Brenzcatechinmonomethylcarbamates in Anwesenheit eines sekundären Amins HNR$_2$ durchführt, wobei R einen in α-Position verzweigten Alkylrest oder einen cycloaliphatischen Rest bedeutet, wie z. B. Diisopropylamin, Dicyclohexylamin oder N-Isopropyl-cyclohexylamin.

Die Ergebnisse sind im Hinblick auf den Stand der Technik überraschend. So ist bekannt, daß bei der Reaktion von Brenzcatechincarbonat mit sekundären Aminen die entsprechenden Brenzcatechincarbamate gebildet werden (Journal f. prakt. Chemie 313, 626 (1971)). Ebenso ist bekannt, daß -Halogenether in hohen Ausbeuten mit sek. Aminen zu den entsprechenden O.N-Acetalen abreagieren (Ann. 702 (1967/68); Chem. Ber. 100 (1967) 7, 2131).

Im Hinblick auf den Stand der Technik hätte man beim erfindungsgemäßen Verfahren in der ersten Stufe eine Reaktion des eingesetzten sek. Amins mit Brenzcatechincarbonat und in der zweiten Stufe eine Alkylierung des Amins durch den 1,2-Dichlorethylmethylether erwartet, d.h. insgesamt mit einer niedrigen Ausbeute und einem heterogenen Produktgemisch gerechnet.

Tatsächlich liefert aber die Umsetzung von Brenzcatechincarbonat und Methylamin bei Anwesenheit von mindestens 1 Mol Äquivalent der obengenannten sek. Amine Brenzcatechinmonomethylcarbamat in einer Ausbeute von 98 - 99 %. Die nachfolgende Umsetzung des Reaktionsgemisches mit 1,2-Dichlorethylmethylethers gelingt wiederum mit einer Ausbeute von über 90 %. Dabei verläuft die Umsetzung im Vergleich zur Reaktion mit tertiären Aminen wesentlich schneller, erfahrungsgemäß um des Acht- bis Zehnfache, mit besserer Selektivität und damit verbunden erhält man das Endprodukt in höherer Reinheit und Stabilität.

Die Umsetzung der ersten Stufe verläuft bei einer Temperetur von -20°C zu +80°C, vorzugsweise 0°C bis +40°C und in der zweiten Stufe bei einer Temperatur von 0°C bis +80°C, vorzugsweise +20°C bis +40°C mit ausreichender Geschwindigkeit.

Als Lösungsmittel werden chlorierte Kohlenwasserstoffe wie Dichlormethan, Dichlorethan, Dichlorethylen oder sogenannte aprotisch-dipolare Lösungsmittel eingesetzt. Solche aprotisch-dipolare Lösungsmittel, wie Tetrahydrofuran, Dimethylformamid oder -sulfoxid sind jedoch im allgemeinen kostspieliger.

Als sekundäre Amine lassen sich die Amine der allgemeinen Formel H-N(R)$_2$ verwenden, bei denen R einen in α-Position verzweigten Alkylrest oder einen cycloaliphatischen Rest bedeutet. Die Reste R können gleich oder verschieden sein. Beispielsweise sind Diisopropylamin, Dicyclohexylamin, Dicyclopentylamin, Cyclohexylisopropylamin, Di-sek.-butylamin geeignet. Die zugesetzte Menge an sekundärem Amin beträgt zweckmäßig 1 bis 2, vorzugsweise 1,2 - 1,5 mol bezogen auf 1 mol Carbonat, d.h. man verwendet vorzugsweise einen stöchiometrischen Überschuß an Amin.

Zweckmäßig legt man Brenzcatechincarbonat zusammen mit dem sek. Amin in dem gewünschten Lösungsmittel vor und führt unter kräftiger Durchmischung (Rückvermischung) Methylamin allmählich zu. Vorteilhaft bei technischer Ausführung kann man eine Lösung von Brenzcatechincarbonat in sek. Amin in einer Umlaufapparatur oder einem Reaktionsrohr kontinuierlich mit Methylamin zur Reaktion bringen. Brenzcatechincarbonat und sek. Amin können auch jeweils in Lösung zugeführt und mit Methylamin zusammen zur Reaktion gebracht werden. Das Reaktionsgemisch wird anschließend unter kräftiger Durchmischung mit 1,2-Dichlorethylmethylether versetzt. Die zugesetzte Menge beträgt zweckmäßig 1 bis 2, vorzugsweise 1,2 bis 1,4 mol bezogen auf 1 mol Carbonat. Die Umsetzung in der zweiten Stufe kann dann in einem System mit unterbrochener Rückvermischung oder ohne Rückvermischung erfolgen.

Beim Arbeiten in chlorierten Kohlenwasserstoffen, wie z. B. Methylenchlorid, läßt sich das gewünschte Produkt aus dem Reaktionsgemisch gewinnen, indem man zunächst das gebildete Aminhydrochlorid extraktiv mit Wasser entfernt und anschließend das Lösungsmittel verdampft. Vorteilhaft bei technischer Ausführung kann der Wirkstoff aus der von Aminhydrochlorid befreiten Lösung durch Zugabe eines umpolaren Kohlenwasserstoffs wie z. B. n-Hexan oder n-Heptan ausgefällt und anschließend durch übliche Methoden wie durch Filtrieren oder Zentrifugieren isoliert werden. Beim Arbeiten in aprotisch-dipolaren Lösungsmittel wie DMF kann der Wirkstoff durch Zugabe von Wasser ausgefällt und wie gezeigt isoliert werden.

## Beispiel

Einer Umlaufapparatur mit einem Fassungsvermögen von 250 ml wurde eine Lösung aus jeweils 2,3 l Methylenchlorid, 550 g Brenzcatechincarbonat und 600 g Diisopropylamin mit einer Zulaufgeschwindigkeit von 2 100 g/h (ca. 1,8 l) und gleichzeitig über einen zweiten Zulauf 67 g Methylamin pro Stunde zugeführt. Die Temperatur wurde bei 28 - 32°C gehalten. Die abfließende Lösung wurde in eine aus zwei Rührkolben mit einem Fafssungsvermögen von je etwa 2 l bestehende Rührkesselkaskade geleitet, wo sie bei 30 bis 35°C mit 330 Gramm 95prozentigem 1,2-Dichlorethylmethylether pro Stunde versetzt wurde. Der Überlauf bzw. der Füllstand der Kaskade waren so eingestellt, daß die mittlere Verweilzeit der Lösung 1,5 Stunden betrug. Aufarbeitung: Die Lösungsmenge von einer Stunde, ca. 2 l, wurde mit 1,5 Liter 0,15prozentiger wäßriger Ameisensäure und anschließend mit 2 Liter 0,6prozentiger Natronlauge extrahiert. Die Methylenchloridphase wurde anschließend bei 15°C unter kräftigem Rühren mit insgesamt 14 l n-Heptan versetzt, das ausgefallene Produkt abgesaugt und getrocknet. Man erhielt 550 g o-(1-Methoxy-2-chlor)ethoxyphenyl-N-methylcarbamat (92 % bezogen auf eingesetztes Brenzcatechincarbonat). Schmelzpunkt: 80°C; Gehalt nach HPLC: 98,6 %.

## Patentansprüche

1. Verfahren zur Herstellung von o-(1-Methoxy-2-chlor)-ethoxyphenyl-N-methylcarbamat durch Umsetzung von Brenzcatechincarbonat mit Methylamin und anschließender Umsetzung des entstandenen Brenzcatechinmonomethylcarbamates mit 1,2-Dichlorethylmethylether in Gegenwart eines Amins, <u>dadurch gekennzeichnet</u>, daß man sowohl die Umsetzung von Brenzcatechincarbonat mit Methylamin als auch die nachfolgende Umsetzung des Brenzcatechinmonomethylcarbamates in Gegenwart eines sekundären Amins $HNR_2$ durchführt, wobei R einen in $\alpha$-Position verzweigten Alkylrest oder einen cycloaliphatischen Rest bedeutet.

2. Verfahren nach Anspruch 1, <u>dadurch gekennzeichnet</u>, daß man die Umsetzung in Gegenwart eines stöchiometrischen Überschusses an sekundärem Amin, bezogen auf Brenzcatechincarbonat vornimmt.

3. Verfahren nach Anspruch 2, <u>dadurch gekennzeichnet</u>, daß der Überschuß bis zu 1 mol/mol beträgt.

4. Verfahren nach Anspruch 1, <u>dadurch gekennzeichnet</u>, daß man die Umsetzung fortlaufend in der Weise vornimmt, daß die erste Stufe unter Rückvermischung und die zweite Stufe in einer Rührkesselkaskade oder nach anfänglicher Rückvermischung in einem Rohrreaktor abläuft.

## Claims

1. A process for the preparation of o-(1-methoxy-2-chloro)-ethoxyphenyl N-methylcarbamate by reacting pyrocatechol carbonate with methylamine and then reacting the resulting pyrocatechol monomethylcarbamate with 1,2-di-chloroethyl methyl ether in the presence of an amine, wherein both the reaction of pyrocatechol carbonate with methylamine and the subsequent conversion of the pyrocatechol monomethylcarbamate are carried out in the presence of a secondary amine $HNR_2$ where R is alkyl which is branched in the $\alpha$-position or is a cycloaliphatic radical.

2. A process as claimed in claim 1, wherein the reaction is carried out in the presence of a stoichiometric excess, based on pyrocatechol carbonate, of a secondary amine.

3. A process as claimed in claim 2, wherein the excess is not more than 1 mole per mole.

4. A process as claimed in claim 1, wherein the reaction is carried out continuously in such a way that the first stage takes place with back-mixing and the second stage takes place in a stirred kettle cascade or, after initial back-mixing, in a tube reactor.

## Revendications

1. Procédé de préparation de carbamate de o-(1-méthoxy-2-chloro)-éthoxyphényl-N-méthyle par réaction de carbonate de pyrocatéchine avec une méthylamine, suivie de la réaction du monométhylcarbamate de pyrocatéchine obtenue avec de l'éther 1,2-dichloroéthylméthylique en présence d'une amine, caractérisé par le fait que l'on effectue aussi bien la réaction du carbonate de pyrocatéchine avec la méthylamine que la réaction suivante du monométhylcarbamate de pyrocatéchine en présence d'une amine secondaire HNR$_2$, R représentant un reste alkyle ramifié en position $\alpha$ on un reste cycloaliphatique.

2. Procédé selon la revendication 1, caractérisé par le fait que l'on opère la réaction en présence d'un excès stoechiométrique d'amine secondaire, par rapport au carbonate de pyrocatéchine.

3. Procédé selon la revendication 2, caractérisé par le fait que l'excès peut aller jusqu'a 1 mole/mole.

4. Procédé selon la revendication 1, caractérisé par le fait que l'on opère la réaction en continu de manière que la première étape se déroule avec mélange en retour et la deuxième étape dans une cascade de chaudières à agitateur ou, après mélange en retour initial, dans un réacteur tubulaire.